Europäisches Patentamt

(19) European Patent Office

Office européen des brevets

(11) Publication number: **0 454 366 A1**

# EUROPEAN PATENT APPLICATION

(21) Application number: **91303506.9**

(51) Int. Cl.⁵: **A23L 1/275, A23J 7/00**

(22) Date of filing: **19.04.91**

(30) Priority: **21.04.90 GB 9009019**

(43) Date of publication of application:
**30.10.91 Bulletin 91/44**

(84) Designated Contracting States:
**AT BE CH DE DK ES FR GB GR IT LI LU NL SE**

(71) Applicant: **UNITED BISCUITS (UK) LIMITED**
**Grant House, Syon Lane**
**Isleworth Middlesex TW7 5NN (GB)**

(72) Inventor: **Hammond, Eugene William**
**"Aisling", Warkton**
**Kettering, Northampton, NN16 9XG (GB)**
Inventor: **Hicks, Ruth Marian**
**"Wood End", Shirwell's Hill, Goring Heath**
**Reading, Berkshire, RG18 7SP (GB)**
Inventor: **Elsheshtawy, Sally**
**21 Bourne Court, South Approach**
**Station Ruislip, Middlesex HA4 6SW (GB)**

(74) Representative: **Ackroyd, Robert et al**
**W.P. THOMPSON & CO. Eastcheap House**
**Central Approach**
**Letchworth, Hertfordshire SG6 3DS (GB)**

(54) **Fat-based food products.**

(57)  The colouring composition comprises liposomes containing entrained colouring material, the liposomes being dispersed in a fat-based edible medium, for example a biscuit cream. The liposomes are preferably formed from a mixture of hardened and unhardened lipids, preferably lecithin. The entrained colouring material is an aqueous colour phase containing a natural, nature-identical or other coloured material. The coloured biscuit cream is prepared by first combining fat and sugar and then adding a blend of hardened and unhardened lecithins and the aqueous colour phase, in which liposomes containing the coloured material are formed.

EP 0 454 366 A1

This invention relates to fat-based food products.

It is often desired in the manufacture of fat-based (which term is to be understood in this specification and its claims to include also oil-based) food products such as white fat confections and biscuit fillings to disperse a colouring material throughout the fat-based food product. In the past, this has been achieved by the formation of an emulsion of the colouring material in the fat-based medium. Such emulsions typically comprise water droplets of about 0.5 μm in diameter. The emulsions however often contain at least some droplets of very much larger diameter (up to several tens of micrometers); some droplets could thus have volumes which are up to $10^4$ or more times larger than the smallest droplets. Thus, even if the number of large droplets is small, their total volume is a large proportion of the dispersed colouring.

Water-based droplets of the sizes mentioned in the preceding paragraph lead to particular problems in fat confections and in cream and filled biscuits, where a coloured, fat-based medium is often in contact with relatively dry biscuit material, for example as in a cream sandwich biscuit. In these circumstances, there is an increasing tendency with increasing droplet size for water droplets to migrate from the filling into the biscuit material, to the detriment of the eating quality of both the biscuit and the filling.

The present invention provides an edible composition comprising a fat-based edible medium and, dispersed therein, liposomes containing entrained colouring material.

The invention also provides, for use in the production of such an edible composition, liposomes containing entrained colouring material. The liposomes may be in aqueous suspension or, preferably, may be dried to form a solid phase, for example a paste.

Further, the invention provides a method of colouring a fat-based edible medium, the method comprising forming liposomes in the presence of water-soluble colouring material, in order to entrain colouring material within the liposomes, and dispersing the liposomes with colouring material entrained therein in the fat-based medium.

Throughout this specification and its claims, the term "colouring" is to be construed to cover those materials which impart colour but have no associated flavour.

In the product, also provided by the invention, of this process, the colouring material may remain occluded by the liposomes, or may be released upon breakdown of the liposomes. If this latter occurs, the product will contain the colouring material dispersed in the fat-based medium as droplets of very much smaller mean size than those formed by the known emulsion processes.

The invention thus takes advantage of the known property of lipids such as lecithin to form vesicles known as liposomes which have the ability to occlude or sequestrate relatively large volumes of aqueous materials whilst being themselves compatible with fat-based environments.

The diameter of the liposomes of the product and composition of the invention is advantageously less than 300 nm and preferably less than 100 nm. Diameters as small as 50 nm and even 25 nm are however possible. Larger diameters than 300 nm are also possible in appropriate circumstances. In most cases however the major part of the liposomes will have diameters within the range 50 to 300 nm.

The very small particle sizes of the colouring material, either as dispersed droplets of occluded by the liposomes, can give rise to a wide range of coloured fat-based coatings, fillings, and laminated or layered products, or combinations of these, using permitted food colourants where the colourants will not migrate between adjacent layers and a sharply-defined colour boundary will be preserved. In such systems it is desirable that the fat contents of the layers be the same compositions, so that inter-layer fat migration is prevented. It is also found that the water-migration problems referred to above can be avoided by use of liposome-based systems, where the very much smaller colouring droplets have a much reduced tendency to migrate into the dry, biscuit material.

The edible media to which the present invention relates are however not restricted to white fat confectionery and biscuit fillings: the invention also finds application in a wide variety of edible products into which the introduction of colouring is desired. Examples of products into which the liposome compositions may be introduced include the following: fresh cream, doughs, fats (for example for baked goods), dressings, oils, mayonnaise, spreads, fondants, mallows, dips (sweet and savoury), ambient-stable dry mixes, icing, glazings, coatings and toppings, including biscuit coatings and toppings, desserts, fillings for cakes and other baked goods, in addition to the white fat confectionery and biscuit fillings already mentioned.

Most of the products mentioned are ones into which the liposome composition would be introduced in a post-preparation step. However, in some cases, for example dough fats, the liposomes would be present at the time of baking of the dough and would be released with advantageous effect, providing expanded volume due to generation of steam.

Lecithin (i.e. phosphatidyl choline) preferably forms a major constituent of the lipid component of the liposomes of the present invention. The liposomes may however be formed in part from other lipids and, indeed, in practice lecithins of the purity grades lower than the highly-purified pharmaceutical grade lecithins (containing 90% or more phosphatidyl choline) are conveniently used. Food grade lecithins having a lecithin content of from about 80% upwards are accordingly suitable. The non-lecithin content of such lecithin compositions is constituted for the most part by other lipids, for example other phospholipids, such as phosphatidyl ethanolamine and lysophosphatidyl choline, and neutral lipids.

It is found that the water-migration problems referred to above can be avoided by use of liposome-based systems, where the very much smaller colouring droplets have a much reduced tendency to migrate into the dry, biscuit material.

The lipids used may be fully hardened lipids, or may be mixtures of hardened and unhardened lipids, some hardened lipid however always being present. Typical mixtures of hardened and unhardened lipids are ones in which the weight ratio of hardened to unhardened lipids being preferably in the range 1:1 to 4:9, the proportion of unhardened lipids preferably being such that the ratio is no greater than 5:8 and more preferably no greater than 6:7.

In this specification, the term "hardened lipid" means a lipid which is substantially fully hydrogenated, that is having an iodine value at or close to the minimum possible value of unity. It is of course necessary that, after hardening of unhardened lipids, a purification step be carried out in order to remove any remaining hardening catalyst or other undesirable impurity. Whilst unhardened lipids are often semi-liquid in nature, hardened lipids are usually solids which can, for example, be milled into a fine powder.

The source of the lipids used in the formation of compositions according to the present invention can be any suitable source compatible with the edible product applications envisaged. One particularly preferred source is soy bean lecithin which allows a product entirely of vegetable origin to be formed. Other sources include egg lecithin and other lecithins.

Suitable commercially-available substantially-pure soy bean lecithin fractions include "Schonat 80" and "Phospholipon 90", both available from Nattermann Phospholipid GmbH (a part of the Rhône-Poulenc Group) and both unhardened lecithins, "Phospholipon 90H" which is available from the same source and is hardened, and "Epicuron 200SH" which is also hardened and is available from Lucas Meyer GmbH of Hamburg. A commercially-available soy bean lecithin containing significant quantities of other lipids but still meeting the criteria for use in a food product is "Bolec FS" which is available from Unimills of Germany. The latter product requires purification before use, for example by acetone precipitation, the resulting product still retaining its non-lecithin phospholipid component but being perfectly adequate for food use.

The entrained colouring material of the liposomes of the invention can be any natural or synthetic (nature-identical or otherwise) colouring which is compatible with the material from which the liposomes are formed.

The entrained material constituting the colour phase will contain an amount of coloured material appropriate to the intensity of the colour and the desired colour level in the final product. The entrained aqueous phase will accordingly usually be no more than 50% by weight coloured material, preferably no more than 25% by weight coloured material and most often no more than 5% by weight coloured material. A typical entrained colour phase is therefore up to 5% coloured material with the balance water.

The final concentration of colourant in the fat-based edible medium (e.g. butter cream) is preferably in the range 0.5 to 1.0% by weight of the medium.

The colours used can be virtually any provided it has no associated flavour.

The liposomes of the present invention can be made by any of the known techniques. This includes hand shaking, homogenisation, ether or alcohol vaporisation techniques, sonication techniques and techniques involving microfluidsation, such as described by H Talsma et al in Drug Development and Industrial Pharmacy 15 (2), 197-207 (1989). The lecithin on which the liposomes is based is preferably pure lecithin but commercially-available lecithin fractions can be used instead. The colouring material can be incorporated into the liposomes simply by its inclusion at an appropriate concentration in the aqueous phase in which the liposomes are formed by whichever method is chosen. After preparation, the temperature of the liposomes is maintained sufficiently low, for example below 30°C and, in any event below their transition temperature, preferably at least 5°C therebelow, to maintain their stability.

Formation of the liposome colouring material into a non-aqueous dispersion allows the material, for example in the form of a paste or a dry powder or a fat or oil dispersion (which may be liquid), to be stored and/or transported before its addition to the fat-based product, in which the liposome-based colouring compositions can be dispersed by mixing of the fat-based product and the solid colouring composition, if necessary after warming of the latter. This gives rise to particular advantages compared with the known emulsion techniques where the emulsions must be formed on-site with the food product and are not reliably shelf-stable.

A typical liposome colouring composition can accordingly be prepared as follows:

The following ingediends are placed in a wide-necked vessel, here a 100cm³ beaker:

| Phosopholipon 90 | 18g |
| Phosopholipon 90H | 8g |
| Aqueous colouring composition (colouring + water) | $14cm^3$ |

The ingredients are mixed under high shear conditions using an Ultra Turrax or a Silverson mixer for 5 min ensuring that the mixer probe is brought into contact with all the ingredients so they are thoroughly mixed. The temperature of the mix is maintained throughout at a temperature no higher than 30°C. The resulting product had a highly-whipped texture not unlike a meringue mixture.

To form a biscuit cream suitable for filling sandwich biscuits, the liposome colouring material is mixed with sugar and fat pre-mixed in the bowl of a Kenwood plenetary mixture and the mixture blended for up to 5 min, again not allowing the temperature to exceed 30°C. The resulting product is a coloured "butter" cream which can be stored or can be used immediately. Sensory testing procedures have shown that products with stable colours over periods of up to 9 months can be prepared. Tests have also shown that cream sandwich biscuits containing the product can be stored for up to 6 months without any sensorily-detectable softening of the biscuit shells taking place.

EXAMPLES 1 to 10

The invention will now be described further by the following specific examples.

In each example, 1 kg of a typical "butter" cream suitable for use in a sandwich biscuit was prepared by pre-mixing the following ingredients in a Kenwood mixer as described in the general method above.

refined icing sugar 600g
BCF3 cream fat 400g

The cream fat used had a typical solid fat index, as measured on a Brücker "Minispec" pulsed NMR spectrometer as follows:

| N20 | 46-56 |
| N25 | 10-20 |
| N30 | 1-6 |
| N32.5 | 1-3 |
| N35 | 0-1 |

To this pre-mix were added respective liposome colouring compositions prepared as described in the general method above, the compositions varying from example to example as shown in Table 1, the amount of diluting water added to the Colorome Yellow being adjusted if necessary to obtain a concentration of the colourant in the final coloured butter cream of 0.5 to 1.0% by weight. Table 1 also gives the total weight of liposome colouring composition added to 0.5 kg of "butter" cream prepared in each case as described above.

In further examples, the Phospholipon 90H can be replaced by Epicuron 200SH and/or the Phospholipon 90 replaced by Schonat 80 or by Bolec FS purified as described above.

In still further examples, the colourant used in Examples 1 to 10 is replaced by another colourant such as Colorome Yellow 9095 or Colorome Orange 9904, both available from Butterfields, or Anthocyanin, Beetroot concentrate OF2 red or Curcumin 6308103 y/o, all available from Overseal.

4

TABLE 1

| Example | 1 | 2 | 3 | 4 | 5 | 6 | 7 | 8 | 9 | 10 |
|---|---|---|---|---|---|---|---|---|---|---|
| Hardened Lecithin[1] | 9g | 8g | 7g | 6g | 5.2g | 5g | 6.5g | 7.5g | 4.5g | 4g |
| Unhardened lecithin[2] | 4g | 5g | 6g | 7g | 7.8g | 8g | 6.5g | 5.5g | 8.5g | 9g |
| Colour phase Colorant solution[3] Diluting water | 20 14 6 | 20 14 6 | 20 14 6 | 20 14 6 | 20 14 6 | 20 14 6 | 20 14 6 | 20 14 6 | 20 14 6 | 20 $cm^3$ 14 $cm^3$ 6 $cm^3$ |
| Amount of liposome Colouring added to 0.5kg of butter cream | 17g | 17g | 17g | 17g | 17g | 17g | 17g | 17g | 17g | 17g |

1 Phospholipon 90H or Epicuron 200SH
2 Phospholipon 90 or Schonat 80 or Bolec FS (purified as described above)
3 Colorome Yellow 9040, available from Butterfields

## Claims

1. An edible composition comprising liposomes containing entrained colouring material.

2. A composition according to claim 1, in which lecithin forms a major constituent of the lipid component of the liposomes.

3. A composition according to claim 2, in which lecithin constitutes at least 80% by weight of the lipid component of the liposomes.

4. A composition according to any preceding claim, in which the lipid components of the liposomes comprises hardened and unhardened lipids, the weight ratio of hardened to unhardened lipids being in the range 1:1 to 4:9.

5. A composition according to claim 4, in which the ratio of hardened to unhardened lipids is no greater than 5:8.

6. A composition according to claim 4, in which the ratio of hardened to unhardened lipids is no greater than 6:7.

7. A composition according to any preceding claim, in which substantially all the liposomes have a diameter of less than 300 nm.

8. A composition according to claim 7, in which substantially all the liposomes have a diameter which is also greater than 25 nm.

9. A composition according to any preceding claim, in which the entrained colour phase is no more than 50% by weight coloured material.

10. A composition according to any of claims 1 to 8, in which the entrained colour phase is no more than 25% by weight coloured material.

11. A composition according to any of claims 1 to 8, in which the entrained colour phase is no more than 5% by weight coloured material.

12. A composition according to any preceding claim in the form of a solid.

13. A composition according to claim 12, in the form of a paste or a dry powder.

14. A composition comprising a fat-based edible medium and, dispersed therein, liposomes according to any preceding claim.

15. A composition according to claim 14, in which the edible medium comprises fat and sugar and the composition is suitable for use as a biscuit filling.

16. A composition according to claim 14 or 15, in which the coloured material forms from 0.5 to 1.0% by weight of the edible medium.

17. A biscuit having a filling according to claim 15 or 16.

18. A method of colouring a fat-based edible medium, comprising forming liposomes in the presence of water-soluble colouring material, in order to entrain colouring material within the liposomes, and dispersing the liposomes with colouring material entrained therein in the fat-based medium.

19. A method according to claim 18, in which the fat-based edible medium comprises fat and sugar which are combined with each other separately and subsequently combined with the liposomes containing entrained colouring material.

**20.** A method according to claim 18 or 19, in which the liposomes are formed by mixing lipids and an aqueous colour composition.

**European Patent Office**

**EUROPEAN SEARCH REPORT**

Application Number

EP 91 30 3506

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DATABASE FSTA accession no. 90:3385, DN 90-30-P0101, International Food Information Service (IFIS), Reading, GB; M. EL-SODA et al.: "Temperature sensitive liposomes: a controlled release system for the acceleration of cheese ripening" & Milchwissenschaft 1989, vol. 44, no. 4, pages 213,214 * whole abstract * | 1 | A 23 L 1/275<br>A 23 J 7/00 |
| | | | TECHNICAL FIELDS SEARCHED (Int. Cl.5)<br><br>A 23 L<br>A 23 J |

The present search report has been drawn up for all claims

| Place of search | Date of completion of the search | Examiner |
|---|---|---|
| BERLIN | 12-07-1991 | ALVAREZ Y ALVAREZ C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document